# EUROPEAN PATENT APPLICATION

(11) **EP 1 510 176 A1**
(43) Date of publication of application: **02.03.2005**
(21) Application number: 04020417.4
(22) Date of filing: 27.08.2004
(51) Int. Cl.: A61B 17/11

(54) **Blood vessel anastomosing appliance**

(30) Priority: 29.08.2003 JP 2003307375; 12.03.2004 JP 2004070551
(71) Applicant: TERUMO KABUSHIKI KAISHA, Tokyo (JP)
(72) Inventor: Kato, Yukitoshi, Ashigarakami-gun Kanagawa (JP)
(74) Representative: Casalonga, Axel

(57) **Abstract**

A blood vessel anastomosing appliance includes a pair of rings to be inserted in two blood vessels, respectively, inside incisions formed in the blood vessels, and threads spanningly laid between the rings to connect them to each other. The blood vessel anastomosing appliance is so configured that, by pulling the threads in the exterior of the blood vessels, the rings are drawn closer to each other, and edge portions of the incisions in the two blood vessels are clamped between the rings. The blood vessel anastomosing appliance further includes fastening members. The fastening members function as a holding unit for holding the condition where the rings have been drawn close to each other, by fastening outer circumferential portions of the rings through the medium of vessel walls of the blood vessels.

## Description

### BACKGROUND OF THE INVENTION

The present invention relates to a blood vessel anastomosing appliance used for making a blood vessel anastomosis.

In a blood vessel anastomotic procedure such as the coronary bypass, for example, in the case of joining the internal thoracic artery and the left coronary artery, the incised left coronary artery and an end of the internal thoracic artery are anastomosed by use of a thread. While the procedure of passing a needle through the coronary artery portion for anastomosis depends on the skill of the surgeon, it is very difficult to carry out where the blood vessel diameter of the coronary artery is small, where the view is restricted and, particularly, under the heart beat.

In view of this, for the purpose of easily achieving anastomosis without suture, a blood vessel anastomosing appliance using two rings has been proposed (see, for example, US Patent No. 6,352,543 (Fig. 14)). The blood vessel anastomosing appliance shown in Fig. 14 of US Patent No. 6,352,543 is for anastomosing side portions of blood vessels to each other, and is so configured that the rings inserted into the inside of incisions in two blood vessels are magnetically attracted toward each other, and the blood vessels are joined to each other by the attracting force. It is intended to ensure that, at the anastomosed portion formed in this way, the blood flows between the two blood vessels inside the rings and through the incisions formed in the side surfaces of the blood vessels.

However, the conventional blood vessel anastomosing appliance mentioned above has the following problems. First, in the case of joining the two rings by the magnetic force, it is difficult to accurately position the rings relative to each other, and the rings are liable to be attracted in an offset (staggered) condition. As a result, the joint between the blood vessels may be spoiled. In the second place, even if the blood vessels have been joined, the absence of a means for maintaining the incisions formed in the side surfaces of the blood vessels in a spread condition generates a problem in that the incisions (cuts) are liable to be closed under elasticity of the blood vessel walls, making it impossible to secure a sufficient bloodstream between the blood vessels.

### SUMMARY OF THE INVENTION

Accordingly, it is an object of the present invention to provide a blood vessel anastomosing appliance with which blood vessels can be accurately anastomosed through an easy operation.

In order to attain the above object, according to the present invention, there is provided a blood vessel anastomosing appliance for anastomosing two blood vessels, including: a pair of rings to be inserted in the two blood vessels, respectively, inside incisions formed in the blood vessels; and a thread spanningly laid between the pair of rings to connect the pair of rings to each other. The pair of rings are brought closer to each other by pulling the thread in the exterior of the blood vessels, whereby edge portions of the incisions in the two blood vessels are clamped between the pair of rings.

In the blood vessel anastomosing appliance, preferably, side portions of the two blood vessels are anastomosed.

In the blood vessel anastomosing appliance, the thread, when pulled, may function to widen the incisions formed in side surfaces of the two blood vessels.

In the blood vessel anastomosing appliance, preferably, the rings are roughly rectangular in shape and so applied that major side directions of the roughly rectangular rings coincide with directions in which the incisions are formed.

In the blood vessel anastomosing appliance, the thread may connect the pair of rings to each other at at least two positions.

In the blood vessel anastomosing appliance, preferably, at least one of the pair of rings is provided with a thread passing portion through which the thread moves when the thread is pulled.

In the blood vessel anastomosing appliance, the thread may connect inner circumferential portions of the pair of rings to each other.

The blood vessel anastomosing appliance may further include a holding unit for holding the condition where the pair of rings have been brought close to each other.

In the blood vessel anastomosing appliance just mentioned, the holding unit may function also as an operating member used for pulling the thread.

In the blood vessel anastomosing appliance just mentioned, preferably, the holding unit includes a fastening member for fastening outer circumferential portions of the pair of rings through vessel walls of the blood vessels.

In the blood vessel anastomosing appliance just mentioned, the fastening member may include a hole for passing the thread therethrough.

In the blood vessel anastomosing appliance just mentioned, preferably, the fastening member is comprised of a clip including a pair of pinching portions which can be brought closer to and away from each other, and a fastening force generating portion for elastically urging the pair of pinching portions toward each other.

In the blood vessel anastomosing appliance just mentioned, the fastening force generating portion may include a pair of arm portions of which base end portions are connected to each other and tip end portions support the pinching portions.

In the blood vessel anastomosing appliance just mentioned, preferably, the pair of arm portions intersect each other in respective intermediate portions and are so configured that the pair of pinching portions are opened wider when portions, on the base end side of the intersecting portions, of the arm portions are closed against the elastic force of the fastening force generating portion by pressing from the outside.

In the blood vessel anastomosing appliance just mentioned, one of the pinching portions may include a hole for passing the thread therethrough.
According to the present invention, two blood vessels can be anastomosed by a simple operation to insert a pair of rings respectively into the inside of incisions formed in the two blood vessels to be anastomosed and thereafter pull
the thread connecting the two rings to each other.

In addition, since the pair of rings are drawn closer to each other while being automatically positioned under the tension of the thread, the condition where the pair of rings are in accurate register with each other can be securely obtained by a simple operation.

Besides, the interval between the rings when the two blood vessels are joined to each other can be regulated by the degree of pulling of the thread. Therefore, the interval between the rings can be regulated according to the wall thickness of the blood vessels, so that the anastomosis can be securely attained according to the individual case of the disease.

In this manner, according to the present invention, blood vessels can be accurately anastomosed through a simple operation.

This application claims priority on Japanese patent applications No. 2003-307375 and No. 2004-70551, the entire contents of which are hereby incorporated by reference.

### BRIEF DESCRIPTION OF THE DRAWINGS

The above and other objects, features and advantages of the present invention will become apparent from the following description and appended claims, taken in conjunction with the accompanying drawings, in which:
Fig. 1 is a perspective view of a first embodiment of the blood vessel anastomosing appliance according to the present invention;
Fig. 2 is a perspective view illustrating the procedure of anastomosing blood vessels by use of the blood vessel anastomosing appliance shown in Fig. 1;
Fig. 3 is a perspective view illustrating the procedure of anastomosing blood vessels by use of the blood vessel anastomosing appliance shown in Fig. 1;
Fig. 4 is a cross-sectional view showing an anastomosed portion in the condition where threads are pulled starting from the condition shown in Fig. 3;
Fig. 5 is a plan view of an incision in the condition shown in Fig. 4;
Fig. 6 is a cross-sectional view of the anastomosed portion in the condition where the anastomotic procedure has been completed;
Fig. 7 is a perspective view of a second embodiment of the blood vessel anastomosing appliance according to the present invention;
Fig. 8 is a cross-sectional view showing an anastomosed portion formed by use of the blood vessel anastomosing appliance shown in Fig. 7;
Fig. 9 is a perspective view of a further embodiment of the blood vessel anastomosing appliance according to the present invention; and
Fig. 10 is a perspective view of the further embodiment of the blood vessel anastomosing appliance according to the present invention.

### DESCRIPTION OF THE PREFERRED EMBODIMENTS

Now, the blood vessel anastomosing appliance according to the present invention will be described in detail below, based on some preferred embodiments shown in the accompanying drawings.

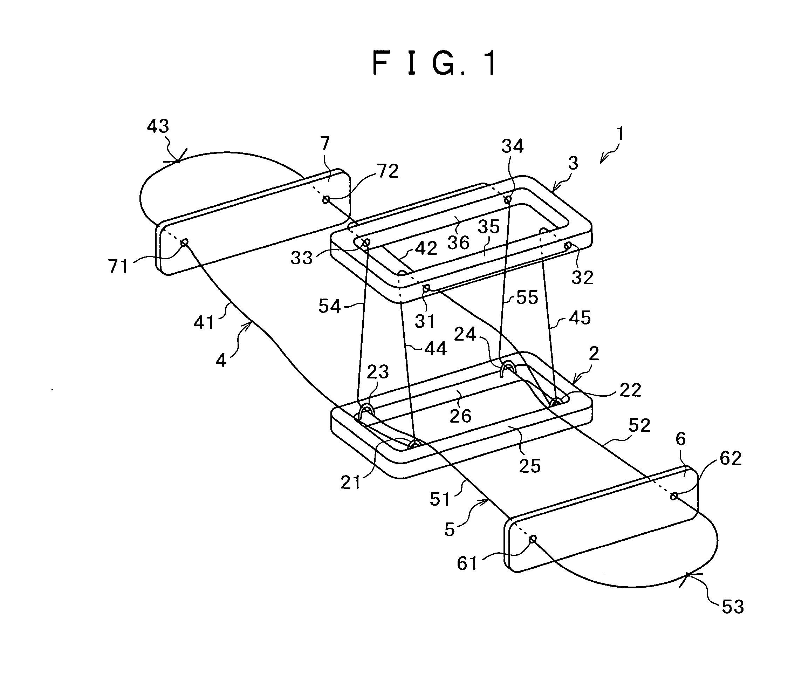

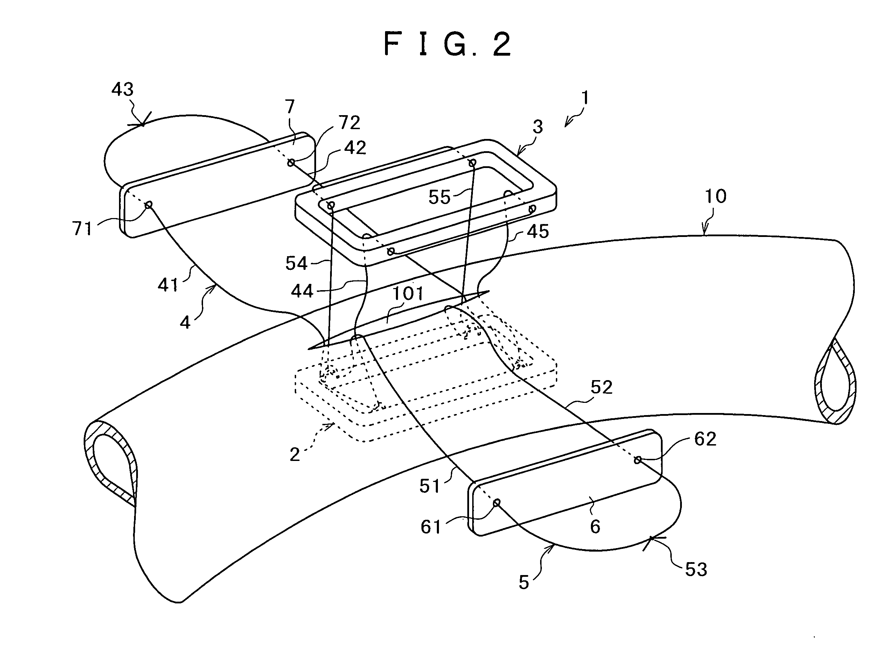

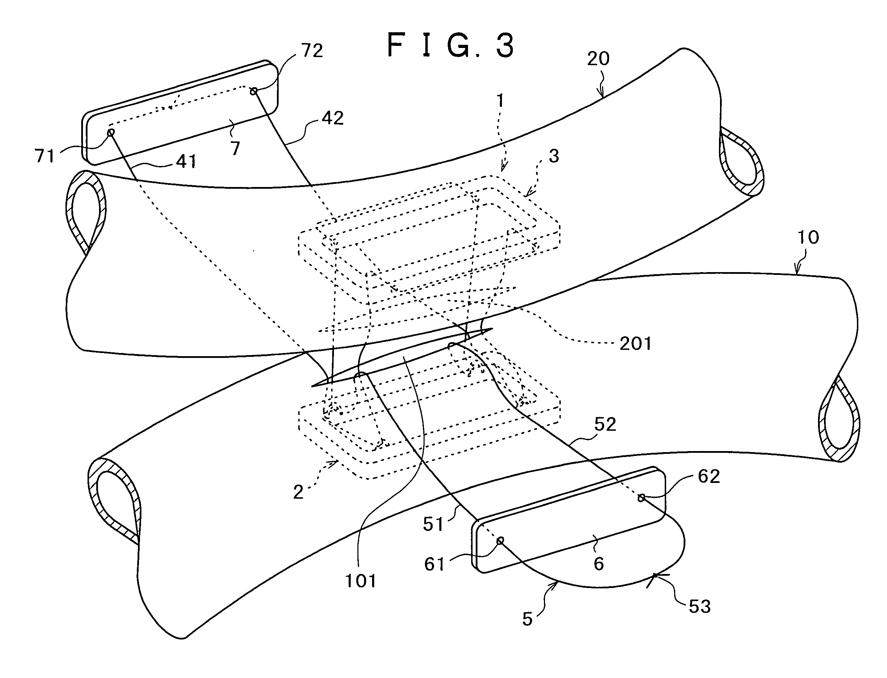

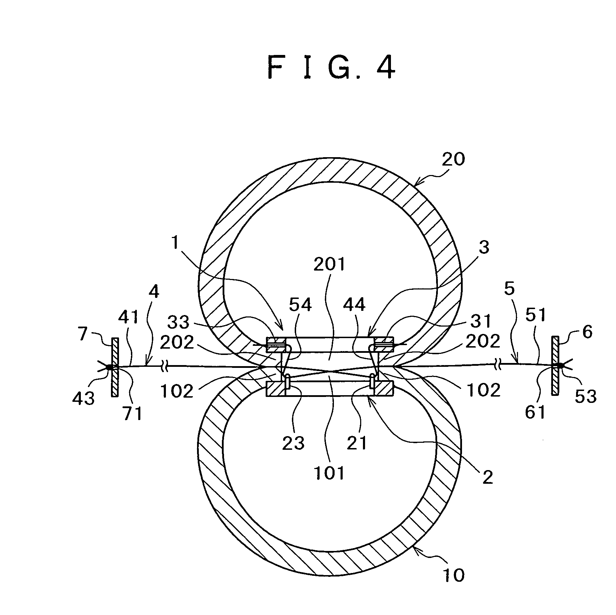

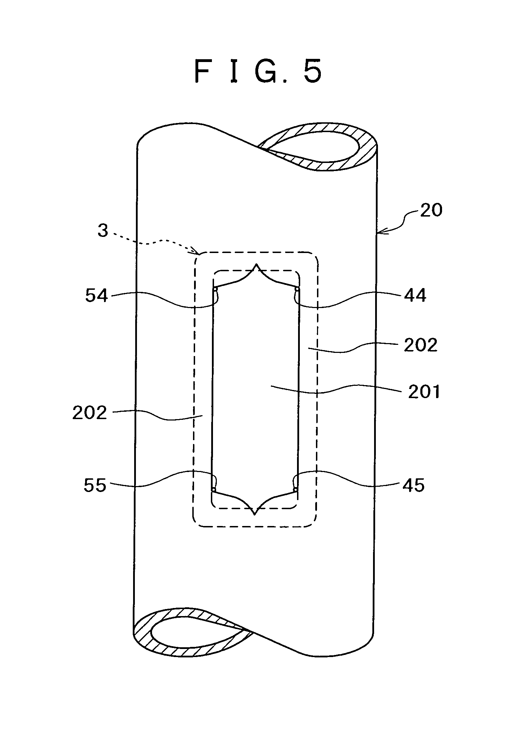

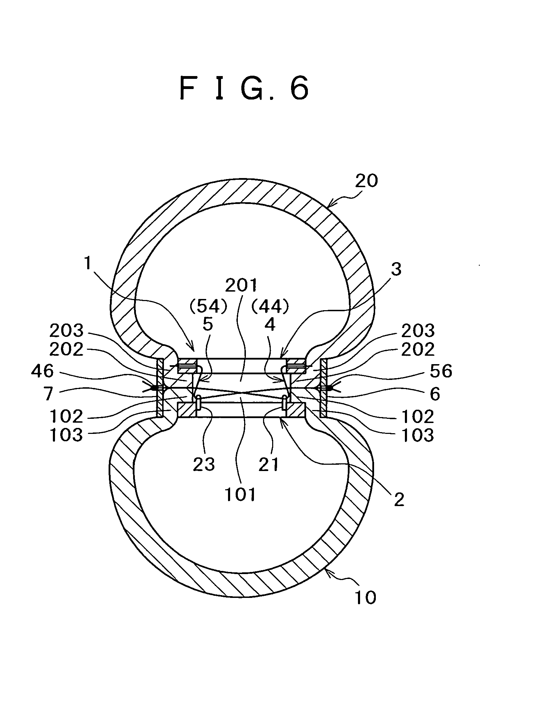

Fig. 1 is a perspective view of a first embodiment of the blood vessel anastomosing appliance according to the present invention; Figs. 2 and 3 are perspective views illustrating the procedure of anastomosing blood vessels by use of the blood vessel anastomosing appliance shown in Fig. 1; Fig. 4 is a cross-sectional view showing an anastomosed portion in the condition where threads are pulled starting from the condition shown in Fig. 3; Fig. 5 is a plan view of an incision in the condition shown in Fig. 4; and Fig. 6 is a cross-sectional view of the anastomosed portion in the condition where the anastomotic procedure has been completed.

The blood vessel anastomosing appliance 1 shown in Fig. 1 is used for anastomosing side portions of two blood vessels 10 and 20 to each other. The blood vessel anastomosing appliance 1 includes a pair of rings (annular bodies) 2 and 3, threads 4 and 5 spanningly laid between the rings 2 and 3 to connect them to each other, and fastening members 6 and 7.

The ring 2 is to be inserted into the inside of an incision (cut) 101 formed in the blood vessel 10 to be anastomosed, and is roughly rectangular frame-like in general shape. The term "roughly rectangular shape" includes the meanings of not only tetragonal shapes but also hexagonal or higher polygonal shapes (e.g., a boat-like hexagonal shape).

The ring 2 is provided with thread passing portions 21 and 22 for passing the thread 4 therethrough, in the vicinity of both ends of an inner circumferential portion of one major side 25 thereof, and is provided with thread passing portions 23 and 24 for passing the thread 5 therethrough, in the vicinity of both ends of an inner circumferential portion of the other major side 26 thereof. In the configuration shown in the figure, these thread passing portions 21 to 24 are each composed of a U-shaped hook.

The ring 3 is to be inserted into the inside of an incision (cut) 201 formed in the blood vessel 20 to be anastomosed to the blood vessel 10, and the ring 3 is roughly rectangular frame-like in general shape, like the ring 2. The ring 3 is provided with holes 31 and 32 for passing the thread 4 therethrough, in the vicinity of both ends of one major side 35 thereof, and is provided with holes 33 and 34 for passing the thread 5 therethrough, in the vicinity of both ends of the other major side 36 thereof. These holes 31 to 34 are formed to penetrate the ring 3 from the inner circumferential surface to the outer circumferential surface thereof.

The material constituting the rings 2 and 3 is not particularly limited. Examples of the material usable include various metallic materials such as stainless steels, aluminum and aluminum alloys, titanium and titanium alloys, nickel-titanium alloys, etc., and various resin materials such as polypropylene, polycarbonate, nylon, polyesters, PTFE, polystyrene, biodegradable resins (e.g., PLLA, PLA, PGA, etc.), acrylic resins, silicones, polyurethane, polyvinyl chloride, copolymers of these resins, etc.

The thread 4 is spanningly laid between the major side 25 of the ring 2 and the major side 35 of the ring 3. Specifically, the thread 4 is passed through the thread passing portion 21 from inside to outside, is spanningly extended to the ring 3, and is passed, from inside to outside, the hole 31 located at a position corresponding to the thread passing portion 21. Further, the thread 4 passed outwards through the hole 31 is extended along the outside surface of the major side 35 to reach the hole 32, is passed through the hole 32 from outside to inside, is spanningly extended to the ring 2, and is passed through the thread passing portion 22 from outside to inside. From the thread passing portions 21 and 22, the thread 4 is further extended outwards beyond the major side 26 on the opposite side.

Similarly, the thread 5 is spanningly laid between the major side 26 of the ring 2 and the major side 36 of the ring 3. Specifically, the thread 5 is passed through the thread passing portion 23 from inside to outside, is spanningly extended to the ring 3, and is passed, from inside to outside, the hole 33 located at a position corresponding to the thread passing portion 23. Further, the thread 5 passed outwards through the hole 33 is extended along the outside surface of the major side 36 to reach the hole 34, is passed through the hole 34 from outside to inside, is then spanningly extended to the ring 2, and is passed through the thread passing portion 24 from outside to inside. From the thread passing portions 23 and 24, the thread 5 is further extended outwards beyond the major side 25 on the opposite side.

With such a configuration, the ring 2 and the ring 3 are connected to each other at four positions by spanning portions 44 and 45 of the thread 4 between the rings 2 and 3 and spanning portions 54 and 55 of the thread 5 between the rings 2 and 3.

In addition, the spanning portions 44, 45, 54 and 55 of the threads 4 and 5 connect the inner circumferential portions of the rings 2 and 3 to each other.

In the blood vessel anastomosing appliance 1 as above, when the rings 2 and 3 are inserted into the inside of the blood vessels 10 and 20 and then portions 41 and 42 of the thread 4 extended to the outside beyond the major side 26 and portions 51 and 52 of the thread 5 extended to the outside beyond the major side 25 are pulled in the exterior, the rings 2 and 3 are drawn closer to each other, and edge portions 102 and 202 of the incisions 101 and 201 are clamped between the rings 2 and 3.

The material constituting the threads 4 and 5 is not particularly limited. Examples of the material usable include various synthetic resin materials such as polypropylene, polyesters, nylon (polyamides), polyethylene, or rayon, various metallic materials such as stainless wires, and various natural materials such as silk.

The fastening members 6 and 7 are rectangular platelike in shape, with the length of the major sides thereof being nearly equal to the length of the major sides of the rings 2 and 3. The fastening member 6 is provided with holes 61 and 62 for passing the thread 5 therethrough, in the vicinity of both ends thereof. Similarly, the fastening member 7 is provided with holes 71 and 72 for passing the thread 4 therethrough, in the vicinity of both ends thereof.

The fastening members 6 and 7 function as a holding unit for holding the condition where the rings 2 and 3 have been drawn close to each other, in such a manner that outer circumferential portions of the rings 2 and 3 are fastened by the fastening members 6 and 7 through vessel walls 103 and 203 of the blood vessels 10 and 20.

The portions 41 and 42 of the thread 4 extended to the outside are passed respectively through the holes 71 and 72 in the fastening member 7, and are then connected to each other by a knot 43. Similarly, the portions 51 and 52 of the thread 5 extended to the outside are passed respectively through the holes 61 and 62 in the fastening member 6, and are then connected to each other by a knot 53.

Incidentally, in the present invention, the laying route of the threads 4 and 5 is not limited to the above-described; the threads 4 and 5 may be laid in any manner as long as the function of drawing the rings 2 and 3 closer to each other can be attained.

Besides, while the rings 2 and 3 are connected to each other at four positions by the spanning portions 44, 45, 54 and 55 in this embodiment as above, the number of the positions of connection between the pair of rings by the threads may be at least two, in the present invention. For example, a configuration may be adopted in which the rings 2 and 3 are connected to each other at two positions, i.e., the midpoints of the major sides 25 and 35 are connected to each other and the midpoints of the major sides 26 and 36 are connected to each other by using threads. In addition, the number of the connection positions may be five or more.

Next, one example of the method of using the blood vessel anastomosing appliance 1 will be described in detail.
[1] As shown in Fig. 2, a side surface of the blood vessel 10 is incised along the longitudinal direction of the blood vessel 10, to form the incision 101. Next, the ring 2 is inserted via the incision 101 into the inside of the blood vessel 10. In this case, the ring 2 is so set that the longitudinal direction thereof coincides with the incision direction of the incision 101.
[2] As shown in Fig. 3, a side surface of the blood vessel 20 is incised along the longitudinal direction of the blood vessel 20, to form the incision 201. Next, the ring 3 is inserted via the incision 201 into the inside of the blood vessel 20. In this case, the ring 3 is so set that the longitudinal direction thereof coincides with the incision direction of the incision 201.
[3] In the condition shown in Fig. 3, the portions 41 and 42 of the thread 4 extended to the outside and the portions 51 and 52 of the thread 5 extended to the outside are pulled respectively, upon which, due to the movement of the threads 4 and 5 through the thread passing portions 21 to 24, the spanning portions 44 and 45 of the thread 4 and the spanning portions 54 and 55 of the thread 5 are decreased in length, and the rings 2 and 3 are drawn closer to each other. This results in that, as shown in Fig. 4, the edge portions 102 of the incision 101 and the edge portions 202 of the incision 201 are clamped between the rings 2 and 3.
   When the threads 4 and 5 are pulled starting from the condition shown in Fig. 3, the spanning portions 44, 45, 54 and 55 function to widen the incisions 101 and 201 (see Fig. 5). As a result, the edge portions 102 and 202 of the incisions 101 and 201 are clamped between the rings 2 and 3 in the condition where the incisions 101 and 201 are securely widened (opened), which makes it possible to secure a sufficient bloodstream between the blood vessel 10 and the blood vessel 20. Incidentally, while the incision 201 is shown in Fig. 5, the incision 101 is also widened similarly.
   Particularly, in this embodiment, since the incisions 101 and 201 are widened at four positions by the spanning portions 44, 45, 54 and 55, a larger opening area can be secured.
   In addition, since the spanning portions 44, 45, 54 and 55 connect the inner circumferential portions of the rings 2 and 3 to each other, the edge portions 102 and 202 of the incisions 101 and 201 can be clamped between the rings 2 and 3 more accurately and securely.
   Besides, in this embodiment, as seen from Fig. 5, the rectangular shape of the rings 2 and 3 makes it possible to secure a particularly large opening area of the incisions 101 and 201, so that it is possible to secure a more sufficient bloodstream between the blood vessel 10 and the blood vessel 20.
   At the time of pulling the threads 4 and 5 starting from the condition shown in Fig. 3, the portions 41, 42, 51 and 52 extended to the outer sides may be pulled directly, or the fastening members 6 and 7 may be gripped with tweezers or the like and pulled to the outer sides. In the latter case, the operation is easy to carry out, the operation can be carried out accurately and swiftly, and the threads 4 and 5 can be more securely prevented from being twisted when pulled. Thus, in this embodiment, the fastening members 6 and 7 can be used also as operating members at the time of pulling the threads 4 and 5.
[4] Starting from the condition shown in Fig. 4, the fastening members 6 and 7 are brought toward the inner sides, while keeping in tension the portions 41, 42, 51 and 52 of the threads 4 and 5 extended to the outer sides. In this instance, the two members move along the portions 41, 42, 51 and 52 which are extended to the outer sides and passed through the holes 71, 72, 61 and 62 in the fastening members 7 and 6, respectively. This operation results in that, as shown in Fig. 6, the vessel walls 103 and 203 of the blood vessels 10 and 20 are clamped between the outer circumferential portions of the rings 2 and 3 and the fastening members 6 and 7. When the outer circumferential portions of the rings 2 and 3 have been fastened by the fastening members 6 and 7, the knots 43 and 53 of the threads 4 and 5 are cut, and the threads 4 and 5 are retied so as to be free of slackening, thereby forming new knots 46 and 56 on the outer sides of the fastening members 6 and 7. This results in that the outer circumferential portions of the rings 2 and 3 are fastened by the fastening members 6 and 7 through the vessel walls 103 and 203 of the blood vessels 10 and 20, and the condition where the rings 2 and 3 have been drawn close to each other is maintained. By the above operations, the procedure of anastomosing the blood vessel 10 and the blood vessel 20 to each other is completed.

As has been described above, in use of the blood vessel anastomosing appliance 1, when the operation to pull the threads 4 and 5 as described in the paragraph [3] above is conducted, the rings 2 and 3 are drawn closer to each other while being automatically positioned relative to each other by the tensions of the threads 4 and 5, so that the condition where the rings 2 and 3 are set in accurate register with each other can be securely obtained through an easy operation.

In addition, with the blood vessel anastomosing appliance 1, the interval between the ring 2 and 3 in the condition of Fig. 6 after completion of the anastomosis can be freely regulated by the degree of pulling of the threads 4 and 5. Therefore, the interval between the rings 2 and 3 can be regulated according to the wall thickness of the blood vessels 10 and 20 (the edge portions 102 and 202), so that the anastomosis can be securely attained according to the individual case of the disease.

Besides, since the rings 2 and 3 can be easily drawn away from each other through slackening the threads 4 and 5 when the operation described in the paragraph [3] above is finished; therefore, in the case where the operation should be started over for some reason, the operation can be easily started over.

Incidentally, in the present invention, the holding unit for holding the condition where the rings 2 and 3 have been drawn close to each other is not limited to the fastening members 6 and 7 or the like but may be any one. For example, a clip or clips for fastening by a spring force or a belt-like tie band or bands for fastening by wrapping around may be used in place of the fastening members 6 and 7. Besides, a configuration may be adopted in which the threads 4 and 5 are fixed by a clip or clips (thread-fixing clip or clips) so as not to be slackened after the operation described in the above paragraph [3] is finished.

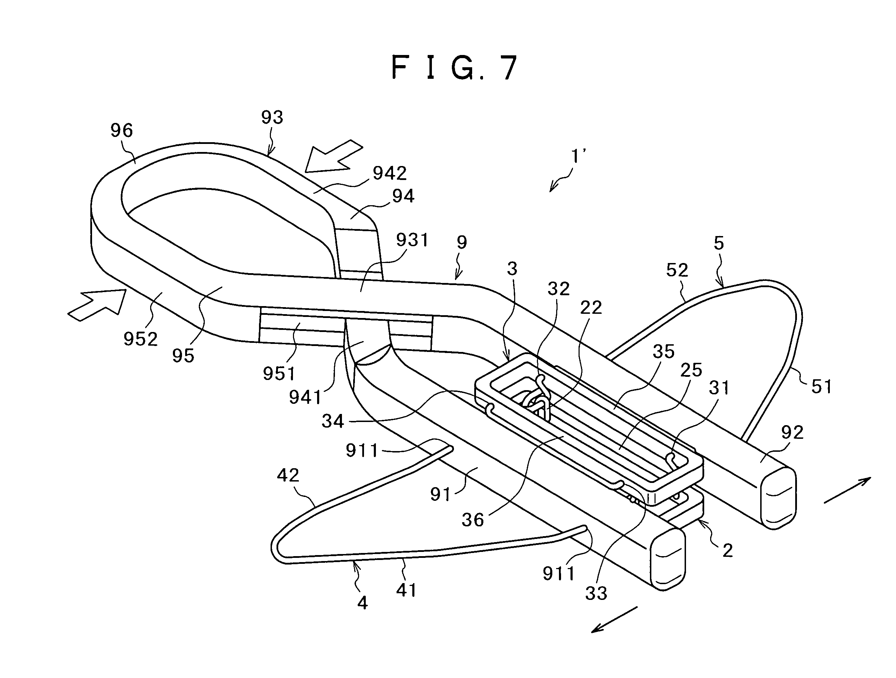

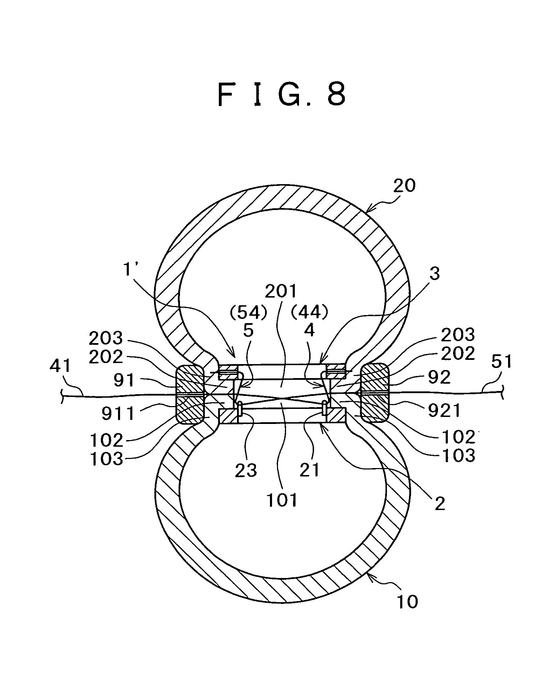

Fig. 7 is a perspective view showing a second embodiment of the blood vessel anastomosing appliance according to the present invention, and Fig. 8 is a cross-sectional view showing an anastomosed portion formed by use of the blood vessel anastomosing appliance shown in Fig. 7.

Now, the second embodiment of the blood vessel anastomosing appliance according to the present invention will be described below referring to these figures. The following description will be centered on the differences from the above-described embodiment, and description of the same items as above will be omitted.

The blood vessel anastomosing appliance 1' in the second embodiment is the same as that in the first embodiment, except for the configuration of the fastening member.

As shown in Fig. 7, the blood vessel anastomosing appliance 1' in the second embodiment includes a clip 9 as the fastening member.

The clip 9 is composed of a pair of pinching portions 91 and 92 which can be brought closer to and away from each other (can be closed and opened), and a fastening force generating portion 93 for urging the pinching portions 91 and 92 toward each other.

The pinching portions 91 and 92 are rod-like in shape, and the length thereof is nearly equal to or slightly larger than the length of the major sides 25, 26, 35 and 36 of the rings 2 and 3.

As shown in Fig. 8, the width of the pinching portions 91 and 92 in the thickness direction of the rings 2 and 3 is so set that the vessel walls 103 and 203 of the blood vessels 10 and 20 can be clamped and fastened between the pinching portions 91 and 92 and the major sides 25, 26, 35 and 36 of the rings 2 and 3 in the condition where the edge portions 102 and 202 of the incisions 101 and 201 are clamped between the rings 2 and 3.

The pinching portion 91 is provided with two thread passing holes 911 for respectively passing therethrough the portions 41 and 42 of the thread 4 extended to the outer side. Similarly, the pinching portion 92 is provided with two thread passing holes 921 for respectively passing therethrough the portions 51 and 52 of the thread 5 extended to the outer side. The thread passing holes 911 and 921 are formed to penetrate through the pinching portions 91 and 92, respectively, from the inside surface to the outside surface at a position in the middle of the width (in the thickness direction of the rings 2 and 3) of the relevant pinching portion.

Incidentally, in the configuration shown, the portions 41 and 42 of the thread 4 extended to the outside are connected to each other without making a knot. Similarly, the portions 51 and 52 of the thread 5 extended to the outside are connected to each other without making a knot.

The fastening force generating portion 93 is composed of a pair of arm portions 94 and 95, and a U-shaped connection portion 96 for connecting base end portions of both the arm portions 94 and 95 to each other. A tip end portion of the arm portion 94 is linked with, and supports, a base end portion of the pinching portion 91. A tip end portion of the arm portion 95 is linked with, and supports, a base end portion of the pinching portion 92.

The arm portions 94 and 95 intersect each other in respective intermediate portions (at an intersection 931). The arm portion 94 is provided, in the vicinity of the intersection 931, with a reduced width portion 941 which is smaller in width than the other portions. The arm portion 95 is provided, in the vicinity of the intersection 931, with a slot 951 for passing the reduced width portion 941 therethrough.

By the elastic force of the fastening force generating portion 93 as above, the pinching portions 91 and 92 are urged in the direction of approaching each other (in a closing direction). In other words, the fastening force generating portion 93 functions as a spring for causing the pinching portions 91 and 92 to approach each other (to close).

In the clip 9 as such, the pinching portions 91 and 92 can be spaced away from each other (be opened) by closing pressed portions 942 and 952, which are portions of the arm portions 94 and 95 on the base end side relative to the intersection 931, through pressing them from the outer sides against the elastic force of the fastening force generating portion 93.

The material constituting the clip 9 is not particularly limited. Examples of the material usable include various metallic materials such as stainless steels, aluminum and aluminum alloys, titanium and titanium alloys, nickel-titanium alloys, and cobalt-chromium alloys, and various resin materials such as polypropylene, polycarbonate, nylon, polyesters, PTFE, polystyrene, biodegradable resins (e.g., PLLA, PLA, PGA, etc.), acrylic resin, silicones, polyurethane, polyvinyl chloride, and copolymers of these.

In addition, the component portions (the pinching portions 91 and 92 and the fastening force generating portion 93) of the clip 9 are preferably formed as one body with each other, but they may be configured as a combination of a plurality of component parts.

One example of the method of using the blood vessel anastomosing appliance 1' including the clip 9 as above will be described below.

In the beginning stage, the pressed portions 942 and 952 of the clip 9 are pressed and set in a pinched state by use of a pinching tool (not shown). By this, the pinching portions 91 and 92 are kept in an opened state.

Next, the rings 2 and 3 are inserted through the incisions 101 and 201 into the inside of the blood vessels 10 and 20.

Subsequently, the portions 41 and 42 of the thread 4 which are extended to the outside and the portions 51 and 52 of the thread 5 which are extended to the outside are pulled respectively. This results in that, due to the movement of the threads 4 and 5 through the thread passing portions 21 to 24 and through the thread passing holes 911 and 921, the rings 2 and 3 are drawn closer to each other, and edge portions 102 and 202 of the incisions 101 and 201 come to be clamped between the rings 2 and 3. In this instance, the presence of the thread passing holes 911 and 921 ensures that the positioning of the rings 2 and 3 and the pinching portions 91 and 92 relative to each other is automatically effected by pulling of the threads 4 and 5, so that the operation can be carried out easily and swiftly without needing skill or time for positioning.

Next, the pressing on the pressed portions 942 and 952 by the pinching tool is canceled. This ensures that the pinching portions 91 and 92 are closed (brought closer to each other) by the elastic force of the clip 9, whereby the outer circumferential portions of the rings 2 and 3 are fastened by the pinching portions 91 and 92 through the vessel walls 103 and 203 of the blood vessels 10 and 20, and the condition where the rings 2 and 3 have been drawn close to each other is maintained (see Fig. 8). By the above operations, the procedure of anastomosing the blood vessel 10 and the blood vessel 20 to each other is completed.

In this embodiment, since the clip 9 is used as the fastening member, there is no need for labor and time for tying the portions of each of the threads 4 and 5 together, so that the anastomosis can be achieved more speedily and easily.

Incidentally, the configuration of the clip 9 is not limited to the one shown in the figures, as long as the equivalent effect (function) can be obtained; for example, the intersection 931 may be absent. That is, there may be used a clip such that two arm portions do not intersect each other and are opened and closed simultaneously to two pinching portions. In addition, the connection portion 96 may be absent, and a pair of arm portions may be fixed to each other by a member such as a pin. Further, the fastening force generating portion is not limited to the one in which the fastening force is generated by the elasticity of the arm portions and the connection portion 96 themselves; for example, there may be used a fastening force generating portion which includes an urging member composed of an elastic material, e.g., a rubber, for generating a fastening force.

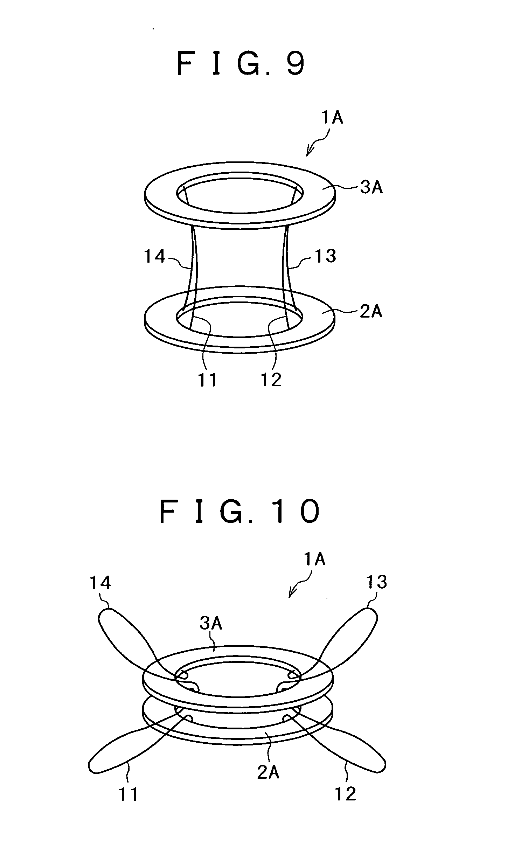

Figs. 9 and 10 are perspective views showing a further embodiment of the blood vessel anastomosing appliance according to the present invention. The further embodiment of the blood vessel anastomosing appliance according to the present invention will be described below, based on these figures. The following description will be centered on the differences from the above-described embodiments, and description of the same items as above will be omitted.

As shown in Fig. 9, the blood vessel anastomosing appliance 1A according to this embodiment includes a pair of rings 2A and 3A. The rings 2A and 3A are circular frame-like in general shape.

Incidentally, the general shape of the rings in the present invention is not limited to the rectangular shape or the circular shape as shown, and may be any shape, for example, a square, an ellipse, or a boat-like shape.

In addition, the blood vessel anastomosing appliance 1A includes threads 11, 12, 13 and 14 spanningly laid between the ring 2A and the ring 3A to connect them to each other. Both end portions of each of these threads 11, 12, 13 and 14 are fixed respectively to the rings 2A and 3A.

As shown in Fig. 10, the blood vessel anastomosing appliance 1A is so configured that when intermediate portions of the threads 11, 12, 13 and 14 are respectively pulled to the outer sides, the rings 2A and 3A are drawn closer to each other, whereby edge portions of incisions in blood vessels can be clamped between the rings 2A and 3A.

While the blood vessel anastomosing appliance according to the present invention has been described referring to the embodiments shown in the figures, the present invention is not limited to the embodiments, and the individual component portions of the blood vessel anastomosing appliance can be replaced by component portions of arbitrary configurations which can display the equivalent functions. Besides, arbitrary structures may be added.

In addition, while the case of side-side anastomosis for anastomosing side portions of blood vessels to each other has been described in the above embodiments, the blood vessel anastomosing appliance according to the present invention is applicable also to the case of end-side anastomosis for anastomosing an end portion of a blood vessel to a side portion of another blood vessel, and to the case of end-end anastomosis for anastomosing end portions of blood vessels to each other.

Incidentally, the term "incision" used herein not only means a cut formed in a side surface of a blood vessel but also includes the concept of an end portion (section portion) of a blood vessel in the cases of end-side anastomosis or end-end anastomosis.

The present invention is not limited to the details of the above described preferred embodiments. The scope of the invention is defined by the appended claims and all changes and modifications as fall within the equivalence of the scope of the claims are therefore to be embraced by the invention.

## Claims

1. A blood vessel anastomosing appliance for anastomosing two blood vessels, comprising:
a pair of rings to be inserted in said two blood vessels, respectively, inside incisions formed in the blood vessels, and
a thread spanningly laid between said pair of rings to connect said pair of rings to each other, wherein
said pair of rings are brought closer to each other by pulling said thread in the exterior of said blood vessels, whereby edge portions of said incisions in said two blood vessels are clamped between said pair of rings.

2. A blood vessel anastomosing appliance as set forth in claim 1, wherein side portions of said two blood vessels are anastomosed.

3. A blood vessel anastomosing appliance as set forth in claim 2, wherein said thread, when pulled, functions to widen said incisions formed in side surfaces of said two blood vessels.

4. A blood vessel anastomosing appliance as set forth in claim 2 or 3, wherein said rings are roughly rectangular in shape and so applied that major side directions of said roughly rectangular rings coincide with directions in which said incisions are formed.

5. A blood vessel anastomosing appliance as set forth in any of claims 1 to 4, wherein said thread connects said pair of rings to each other at at least two positions.

6. A blood vessel anastomosing appliance as set forth in any of claims 1 to 5, wherein at least one of said pair of rings is provided with a thread passing portion through which said thread moves when said thread is pulled.

7. A blood vessel anastomosing appliance as set forth in any of claims 1 to 6, wherein said thread connects inner circumferential portions of said pair of rings to each other.

8. A blood vessel anastomosing appliance as set forth in any of claims 1 to 7, further comprising a holding unit for holding a condition where said pair of rings have been brought close to each other.

9. A blood vessel anastomosing appliance as set forth in claim 8, wherein said holding unit functions also as an operating member used for pulling said thread.

10. A blood vessel anastomosing appliance as set forth in claim 8 or 9, wherein said holding unit comprises a fastening member for fastening outer circumferential portions of said pair of rings through vessel walls of said blood vessels.

11. A blood vessel anastomosing appliance as set forth in claim 10, wherein said fastening member comprises a hole for passing said thread therethrough.

12. A blood vessel anastomosing appliance as set forth in claim 10 or 11, wherein said fastening member is comprised of a clip comprising a pair of pinching portions which can be brought closer to and away from each other, and a fastening force generating portion for elastically urging said pair of pinching portions toward each other.

13. A blood vessel anastomosing appliance as set forth in claim 12, wherein said fastening force generating portion comprises a pair of arm portions of which base end portions are connected to each other and tip end portions support said pinching portions.

14. A blood vessel anastomosing appliance as set forth in claim 13, wherein said pair of arm portions intersect each other in respective intermediate portions and are so configured that said pair of pinching portions are opened wider when portions, on a base end side of the intersecting portions, of the arm portions are closed against the elastic force of said fastening force generating portion by pressing from outside.

15. A blood vessel anastomosing appliance as set forth in any of claims 12 to 14, wherein one of said pinching portions comprises a hole for passing said thread therethrough.
